# EUROPÄISCHE PATENTSCHRIFT

(11) **EP 2 665 934 B1**
(45) Veröffentlichungstag und Bekanntmachung des Hinweises auf die Patenterteilung: **26.11.2014**
(21) Anmeldenummer: 13710339.6
(22) Anmeldetag: 08.03.2013
(51) Int. Cl.: F04B 7/00, F04B 13/00, A61M 5/142

(54) **KOLBENPUMPE; VORRICHTUNG ZUR ZUFÜHRUNG UND DOSIERUNG EINES FLUIDS FÜR MEDIZINISCHE ZWECKE MITTELS KOLBENPUMPE**
PISTON PUMP; DEVICE FOR FEEDING AND METERING A FLUID FOR MEDICAL PURPOSES BY MEANS OF A PISTON PUMP
POMPE À PISTON ; DISPOSITIF PERMETTANT D'ACHEMINER ET DE DOSER UN FLUIDE À FINALITÉ MÉDICALE AU MOYEN D'UNE POMPE À PISTON

(30) Priorität: 19.03.2012 DE 102012102272
(43) Veröffentlichungstag der Anmeldung: 27.11.2013
(73) Patentinhaber: B. Braun Melsungen AG, 34212 Melsungen (DE)
(72) Erfinder: EBERHARD, Dietmar, 79341 Kenzingen (DE)
(74) Vertreter: Winter, Brandl, Fürniss, Hübner, Röss, Kaiser, Polte - Partnerschaft mbB
(86) Internationale Anmeldenummer: PCT/EP2013/054770
(87) Internationale Veröffentlichungsnummer: WO 2013/139630

(56) Entgegenhaltungen:
- WO-A1-2008/086349
- US-A1- 2011 021 990
- US-B2- 7 887 308

## Beschreibung

Die Erfindung betrifft eine Kolbenpumpe zur Förderung eines Fluids, umfassend wenigstens zwei Zylinder mit jeweils einem Kolben, der mittels eines Antriebs innerhalb des zugehörigen Zylinders entlang der Längsachse des Zylinders bewegbar ist, wobei die Zylinder auf einem gemeinsamen Pumpenflansch angebracht sind. In jedem Zylinder bildet sich eine Kammer mit veränderlichem Volumen aus, wenn der zugehörige Kolben im Zylinder bewegt wird.

Die Erfindung betrifft ferner eine Vorrichtung zur Zuführung und Dosierung eines Fluids für medizinische Zwecke, wobei die Vorrichtung eine solche Kolbenpumpe umfasst.

Kolbenpumpen eignen sich insbesondere für den Einsatz in der medizinischen Infusionstechnik. Dort kommen derzeit vorwiegend Schlauch- und Spritzenpumpen zum Einsatz. Schlauchpumpen, die nach dem Peristaltikprinzip arbeiten, werden vor allem dann eingesetzt, wenn größere Flüssigkeitsmengen verabreicht werden sollen. Eine Bereitstellung dieser Flüssigkeitsmengen erfolgt beispielsweise durch Infusionsbeutel. Bei Spritzenpumpen ist das Fördervolumen durch den Spritzenkörper begrenzt und umfasst in der Regel nicht mehr als 50 ml, wobei einige Modelle auch einen Flüssigkeitsvorrat von bis zu 100 ml ermöglichen.

Schlauchpumpen nach dem Peristaltikprinzip sind sehr verbreitet und werden auch ambulatorisch z.B. für die künstliche Ernährung eingesetzt. Aufgrund ihres Pumpprinzips ist ihre Fördergenauigkeit jedoch schlechter als die der Spritzenpumpen. Ferner ist das sichere Verschließen der Ein- und Auslassstutzen von entscheidender Bedeutung, wobei herkömmliche Infusions-Sets, deren Fluidförderfunktion über ein PeristaltikSegment ermöglicht wird, zur Unterbindung eines schwerkraftinduzierten Fluidflusses meist eine sogenannte "Free Flow Klemme" besitzen, die den Schlauch einfach abdrückt.

Bei diesen Einsatzgebieten ist darüber hinaus eine zuverlässige Okklusionserkennung unverzichtbar, da eine nicht erkannte Unterförderung ein hohes medizinisches Risiko darstellen kann. Die Erkennung einer Okklusion erfolgt dabei meist durch eine indirekte Messung des Innendrucks in einem Schlauch, der zur Zuführung einer Flüssigkeit zu einem Patienten dient. Besteht eine Okklusion, erhöht sich beispielsweise stromab der Pumpe der Innendruck im Schlauch, was indirekt gemessen werden kann. Hierzu wird der runde Schlauchquerschnitt oftmals durch eine Vorspannkraft elliptisch verformt und der zu bestimmende Innendruck des Schlauchs erhöht oder vermindert diese Vorspannkraft, die dann mittels eines Kraftsensors ermittelt werden kann. Die DE 38 38 689 C1 offenbart exemplarisch ein derartiges Verfahren zur Druckmessung und Okklusionserkennung.

Beim Einlegen eines Schlauchsets in eine Pumpe muss nach heutigem Stand der Technik daher oftmals das für eine Okklusionssensorik zuständige Schlauchsegment zusätzlich per Hand in spezielle Halterungen eingelegt werden, was nicht nur im Bereich der häuslichen Pflege problematisch sein kann. Nachteilig bei dieser Methode ist ferner, dass die Verformung des Schlauchs in der Regel zu einem über Stunden anhaltenden Kriechvorgang führt. Dieses Kriechen baut Spannungen im Schlauchquerschnitt ab, was zu einer stetigen Änderung der gemessenen Kraft führt. Die durch das Kriechen verursachte und unerwünschte Kraftänderung ist in einer ähnlichen Größenordnung wie der gewünschte Messeffekt durch die Variation des Schlauchinnendrucks und erschwert somit die sichere Erkennung einer Okklusion. Spezielle Elastomere wie beispielsweise Silikone weisen ein deutlich vermindertes Kriechverhalten auf und sind daher als Schlauchsegment für die Okklusionssensorik prädestiniert. Eine Verbindung von Silikon- mit Nichtsilikonmaterialien ist allerdings sehr aufwändig, da prozesssichere Klebungen nicht möglich sind.

Periodisch arbeitende Kolbenpumpen weisen wie Spritzenpumpen eine hohe Fördergenauigkeit auf und können Fluide wie Peristaltikpumpen aus einem austauschbaren Vorratsbehälter ansaugen und weiterfördern. Eine derartige Pumpe ist beispielsweise in der US 7,887,308 B2 beschrieben. Diese offenbart verschiedene Kolbenpumpen mit einer hin- und her rotierenden Ventilscheibe, um die Pumpfunktion zu gewährleisten. Ihre Funktion ist allerdings auf eine alleinige Pumpfunktion beschränkt. Da die Ein- und Auslassverbindungen an der sich hin- und her bewegenden Ventilscheibe angebracht sind, bewegen sich die Verbindungsschläuche ferner mit der Pumpbewegung, wodurch viel Bauraum beansprucht wird.

Darüber lässt sich bei den aus der US 7,887,308 B2 bekannten Pumpen eine notwendige Okklusionssensorik nicht kompakt in den Pumpenkörper integrieren. Bedingt durch drei mechanische Kopplungen für den Antrieb (2 x Kolben und 1 x Ventilscheibe) ist die Antriebsschnittstelle der Pumpe nach US 7,887,308 B2 ferner mechanisch hochgradig überbestimmt, wodurch das Einlegen erschwert wird. Aufwändige und präzise Führungen der Pumpe während des Einlegens bzw. der Entnahme sind für eine sichere und einfache Handhabung zwingend notwendig.

Eine Free Flow-Klemmenfunktion oder weitere Sensorikkomponenten sind bei den Pumpenausführungen der US 7,887,308 B2 ebenfalls nicht integriert. Bei einem nicht in den Pumpenkörper eingelegten Infusions-Set kann daher durch eine druckinduzierte Bewegung der Kolben eine Förderung von einer Zylinderfüllung erfolgen. Im Gegensatz zum gewohnten Befüllen eines Infusions-Sets durch Schwerkraft vor der Therapie (Priming), erlaubt die in US 7,887,308 B2 beschriebe Kolbenpumpe nur eine Befüllung des Infusions-Sets durch Inbetriebnahme der Kolbenpumpe selbst. Werden Infusions-Sets beispielsweise in einer zentralen Klinikeinheit befüllt und sollen anschließend in andere Pumpenantriebe in der Nähe der Patienten eingelegt werden, muss das Befüllen mit einer definierte Kolben- und Ventilscheibenstellung abgeschlossen werden, da sonst ein Einlegen in den anderen Pumpenantrieb nicht möglich ist. Da das Ende einer Befüllung kaum mit dieser Initialposition von Kolben und Ventilscheibe übereinstimmen wird, ist eine Überförderung vonnöten, die zu einem Austritt von Fluiden am patientenseitigen Ende der Infusions-Sets führt.

Benachbarter Stand der Technik ist aus den Druckschriften US 2011/0021990 A1, US 2011/0206545 1 und US 4 854 836 A ebenfalls bekannt.

Aufgabe der Erfindung ist es daher, eine Kolbenpumpe und damit auch eine Vorrichtung zur Zuführung und Dosierung eines Fluids für medizinische Zwecke bereitzustellen, welche sowohl für die Pumpenfunktion als auch für zusätzliche Komponenten wie eine Okklusions- und Ultraschallsensorik eine kompakte Bauweise und einfache Handhabung ermöglicht. Ferner soll eine sichere Absperrfunktion realisierbar sein.

Erfindungsgemäß wird diese Aufgabe durch eine Kolbenpumpe gemäß dem unabhängigen Anspruch 1 gelöst. Vorteilhafte Weiterbildungen dieser Kolbenpumpe ergeben sich aus den Unteransprüchen 2-14. Die Aufgabe wird ferner durch eine Vorrichtung zur Zuführung und Dosierung eines Fluids für medizinische Zwecke gemäß Anspruch 15 gelöst.

Die erfindungsgemäße Kolbenpumpe dient zur Förderung eines Fluids und umfasst wenigstens zwei Zylinder mit jeweils einem Kolben, der mittels eines Antriebs innerhalb des zugehörigen Zylinders entlang der Längsachse des Zylinders bewegbar ist. Dabei sind die Zylinder auf einem gemeinsamen Pumpenflansch angebracht, und in jedem Zylinder bildet sich eine Kammer mit veränderlichem Volumen, wenn der zugehörige Kolben im Zylinder bewegt wird.

Erfindungsgemäß erstreckt sich der Pumpenflansch entlang der Bewegungsrichtung der Kolben, und auf dem Pumpenflansch sind wenigstens ein Einlassstutzen und ein Auslassstutzen angebracht, deren Längsachsen ebenfalls entlang dem Pumpenflansch verlaufen. In einem bevorzugten Ausführungsbeispiel der Erfindung erstreckt sich der Pumpenflansch parallel zur Bewegungsrichtung der Kolben, und die Ein- und Auslassstutzen verlaufen ebenfalls parallel zum Pumpenflansch. Allerdings sind auch Abweichungen von der Parallelität von der Erfindung umfasst, so dass sich der Pumpenflansch auch lediglich in Richtung der Bewegungsrichtung des Kolbens erstrecken kann, während die Ein- und Auslassstutzen ebenfalls in Richtung des Pumpenflansches verlaufen. Die Abweichung von der Parallelität kann beispielsweise in der Größenordnung von 1-20° liegen, ist jedoch nicht auf diese Werte beschränkt.

Auf der den Zylindern abgewandten Seite des Pumpenflansches ist erfindungsgemäß ferner eine zentrale Ventilscheibe angebracht, die an dem Pumpenflansch anliegt und sich bei Förderbetrieb der Kolbenpumpe stetig dreht. Dabei verläuft die Drehachse der Ventilscheibe quer und vorzugsweise senkrecht zum Pumpenflansch. Im Bereich der Einlass- und Auslassstutzen sind jeweils Durchlässe und im Bereich der Zylinder jeweils Zylinderöffnungen in den Pumpenflansch eingebracht, wobei die Ventilscheibe flanschseitig wenigstens zwei Vertiefungen aufweist, von denen eine erste Vertiefung bei Drehung der Ventilscheibe in eine erste Winkelposition mit einer Zylinderöffnung eines ersten Zylinders und einem Durchlass des Auslassstutzens in Deckung ist, während die zweite Vertiefung in dieser ersten Winkelposition mit dem Durchlass des Einlassstutzens und einer Zylinderöffnung in dem zweiten Zylinder in Deckung ist. Die erste Vertiefung ist ferner bei Drehung der Ventilscheibe in eine zweite Winkelposition mit dem Durchlass im Auslassstutzen und einer Zylinderöffnung im zweiten Zylinder in Deckung, während die zweite Vertiefung in dieser zweiten Winkelposition mit dem Durchlass im Einlassstutzen und einer Zylinderöffnung im ersten Zylinder in Deckung ist.

Durch diese Anordnung von Zylindern, Stutzen und einer Ventilscheibe an einem gemeinsamen Flansch lässt sich für die erfindungsgemäße Kolbenpumpe eine sehr kompakte Bauweise erreichen, ohne dass unbewegliche oder bewegliche Bauteile zu viel Bauraum benötigen. Dies ist insbesondere dann der Fall, wenn die Längsachsen der Zylinder und/oder die Längsachsen der Einlass- und Auslassstutzen in etwa in die gleiche Richtung oder sogar parallel zueinander verlaufen. Ferner sind die Bewegungsrichtungen der Kolben vorzugsweise gleich.

Im Gegensatz beispielsweise zu der aus der US 7,887,308 B2 bekannten Pumpe liegt ferner die Ebene der Ventilscheibe im Wesentlichen parallel zur Hauptmontageebene der Pumpe. Da die Ventilscheibe beim Einlegen mechanisch an den Antrieb angekoppelt werden muss, ergeben sich bessere mechanische Gestaltungsmöglichkeiten, die auf einer verbesserten ergonomischen Handhabung beruhen. Beispielsweise entfällt ein komplexer Doppel-Exzenterantrieb zur Funktionssteuerung der Pumpe, wie er nach der US 7,887,308 B2 vorgesehen ist. Auch bewegte Schlauchsegmente können vermieden werden, was eine deutlich einfachere Assemblierung ermöglicht.

Im Vergleich zu Peristaltikpumpen wird darüber hinaus durch das Kolbenpumpenprinzip eine deutlich höhere Genauigkeit erreicht. Insbesondere bei der kurzzeitigen Flusskonstanz, die aus den sogenannten "Trompetenkurven" ersichtlich ist, ist das Kolbenpumpenprinzip dem Peristaltikpumpenprinzip deutlich überlegen. Die Toleranzanforderungen werden durch eine Ventilscheibe, die nur auf einer Seite eine Dichtfunktion hat, ebenfalls deutlich entschärft.

In einem Ausführungsbeispiel der Erfindung sind die Vertiefungen der Ventilscheibe bei Drehung der Ventilscheibe in wenigstens eine dritte Winkelposition mit keiner Zylinderöffnung in Deckung. Vor der Entnahme des Infusions-Sets kann die Ventilscheibe daher durch eine entsprechende motorische Drehung in eine Position gedreht werden, in der analog zu Peristaltik-Infusions-Sets ein kompletter Free Flow-Schutz möglich ist. Vorzugsweise liegt diese wenigstens eine dritte Winkelposition zwischen den beiden zuvor genannten Winkelpositionen, in der die beiden Zylinder ansaugen bzw. ausstoßen.

Vorzugsweise bewirkt der Antrieb sowohl die Bewegung der Kolben, als auch die Drehung der Ventilscheibe. Dabei ist der Antrieb in einem Ausführungsbeispiel der Erfindung ein Exzenterantrieb mit einer Exzenterscheibe und einem die Exzenterscheibe umgebenden Rahmen, wobei der Drehpunkt V der Ventilscheibe von dem Drehpunkt E der Exzenterscheibe abweicht. Ferner bewirkt eine rotatorische Antriebseinheit bei Förderbetrieb der Kolbenpumpe die Drehung der Ventilscheibe, wobei die Antriebseinheit so an die Exzenterscheibe gekoppelt ist, dass sich die Exzenterscheibe mit der Ventilscheibe dreht und an zwei gegenüber liegenden Rahmenflanken an den Rahmen anläuft, wodurch sich der Rahmen in Richtung der beiden Rahmenflanken hin- und her bewegt. Die Kolben sind jeweils so an den Rahmen gekoppelt, dass die Hin- und Herbewegung des Rahmens auf die Kolben übertragbar ist. Auf diese Weise kann sowohl die Bewegung der Kolben, als auch die Drehung der Ventilscheibe kompakt durch einen gemeinsamen Antrieb realisiert werden.

Vorzugsweise ist die Exzenterscheibe dabei lösbar an die Antriebseinheit gekoppelt. Hierfür kann die Exzenterscheibe beispielsweise ringförmig ausgebildet sein und die Antriebseinheit umgeben, wobei die Antriebseinheit an ihrem Außenumfang eine Nase aufweist, während die Exzenterscheibe an ihrem Innenumfang einen Zapfen aufweist, der bei Förderbetrieb der Kolbenpumpe an der Nase anliegt. Wird die Ventilscheibe dann durch die Antriebseinheit rückwärts gedreht, löst sich die Nase vom Zapfen und die aktuelle Stellung der Kolben und der Ventilscheibe kann fixiert werden. So kann die Art des Antriebs vorteilhaft für eine Free Flow-Klemmfunktion genutzt werden.

Optional kann in den Einlassstutzen, den Auslassstutzen und/oder den Pumpenflansch jeweils wenigstens eine Okklusionssensorik integriert sein, die nicht zerstörungsfrei demontierbar ist, wobei der Einlassstutzen, der Auslassstutzen und/oder der Pumpenflansch als Gehäuse der Okklusionssensorik dienen können.

Beispielsweise kann dazu in einem so gebildeten Gehäuse wenigstens eine Aussparung vorgesehen sein, welche dicht durch eine Sensorkomponente abgedeckt wird, die aus einem druckempfindlichen Material besteht. Dabei ist das Material des Gehäuses härter als das der Sensorkomponente, und die Kolbenpumpe weist einen Kraftsensor auf, mit dem druckinduzierte Veränderungen der Sensorkomponente im Bereich der jeweiligen Aussparung messbar sind.

Die Erfindung macht sich somit das Funktionsprinzip einer Druckmembran zunutze, setzt dieses jedoch nicht in einem separaten Bauteil ein, sondern integriert eine entsprechende Sensorkomponente in Gehäuse aus einem harten Material, durch den ein Fluid ohnehin gefördert wird. Dabei basiert die zu integrierende mechanische Sensorkomponente der Okklusionssensorik auf dem Prinzip der Druckmessung im Fluid und wird durch ein elastischen Material realisiert, das sich physikalisch analog einer Druckmembran verhält. Ein Stutzen und/oder der Pumpenflansch selbst bilden dabei eine druckunempfindliche Hartkomponente, die sich bei den auftretenden Druckänderungen nicht verformt. Aufgrund der auftretenden Verformungen der Sensorkomponente als Weichkomponente kann dagegen auf den Innendruck im Stutzen geschlossen werden.

Die Okklusionssensorik kann platzsparend direkt in ein solches Gehäuse integriert werden, was eine sehr kompakte Bauweise ermöglicht. Bei einem Stutzen kann es sich um einen Einlass- und/oder Auslassstutzen handeln, der das Fluid zu einer Pumpe bzw. von dieser zu einem Patienten führt. Die Sensorik kann auf diese Weise Okklusionen vor und/oder hinter einer Pumpe erkennen. Wird der zugehörige Stutzen entsprechend so positioniert, dass er kompakt mit einer Pumpe in einem Gehäuse untergebracht werden kann, wird durch die Okklusionssensorik an diesem Stutzen kaum weiterer Bauraum benötigt.

Die Sensorkomponente ist dabei vorzugsweise ein integraler und nicht demontierbarer Bestandteil eines jeweiligen Gehäuses, so dass sie auch nicht bei Inbetriebnahme der Vorrichtung installiert oder sogar ausgerichtet werden muss. Dies erleichtert die Handhabung der Vorrichtung und vermeidet Einrichtungsfehler und somit auch Messfehler. Vorzugsweise hat der Kraftsensor im Bereich einer Aussparung im Gehäuse Kontakt mit der Oberfläche der Sensorkomponente, wobei der Kraftsensor beispielsweise einen Stempel aufweist, der im Bereich der Aussparung in direktem Kontakt mit der Oberfläche der Sensorkomponente steht. So kann eine Veränderung der Ausdehnung der Sensorkomponente in diesem Bereich gemessen werden.

Ferner besteht die Sensorkomponente zweckmäßigerweise aus einem Elastomer, wobei es sich insbesondere um Silikon oder ein thermoelastisches Elastomer handeln kann. So können die physikalischen Eigenschaften dieser speziellen Elastomere vorteilhaft genutzt werden, was insbesondere ein geringes Kriechverhalten beinhaltet. Eine stoffschlüssige Verbindung von Silikon- und Nichtsilikonmaterialien ist dabei jedoch nicht erforderlich, da für eine dichte Verbindung zwischen Stutzen und Sensorkomponente geeignete Verfahren wie beispielsweise Spritzgussverfahren eingesetzt werden können. So können das jeweilige Gehäuse und die Sensorkomponente in einem Zweikomponentenverfahren hergestellt werden. Alternativ kann die Verbindung zwischen Stutzen und Sensorkomponente auch durch andere Verbindungstechniken hergestellt werden, wobei beispielsweise Steck-, Klick-, Schraub- oder auch Klebeverbindungen in Frage kommen.

In einem Ausführungsbeispiel der Okklusionssensorik ist die Sensorkomponente ein Schlauch, der einen Stutzen formschlüssig umgibt, so dass sie eine Aussparung im Stutzen dicht von außen abdeckt. In einem anderen Ausführungsbeispiel ist dieser Schlauch formschlüssig innerhalb eines Stutzens angebracht, so dass die Sensorkomponente eine entsprechende Aussparung dicht von innen abdeckt. Dabei haben der Stutzen und die Sensorkomponente zweckmäßigerweise einen ähnlichen oder gleichen Querschnitt. Beispielsweise kann ein Schlauch mit rundem Querschnitt formschlüssig in einen runden Stutzen eingebracht werden bzw. diesen umschließen.

Allerdings kann es auch vorteilhaft sein, wenn die Sensorkomponente einen elliptischen Querschnitt hat, wobei eine flache Seite der Sensorkomponente im Bereich der Aussparung angeordnet ist. Dies kann sowohl für innen als auch für außen liegende Sensorkomponenten der Fall sein, wobei der Querschnitt des Stutzens entsprechend angepasst werden kann. Durch diese Form der Weichkomponente hat die Sensorkomponente bereits die für die Innendruckmessung notwendige elliptische Verformung, so dass unerwünschtes Kriechverhalten bei thermoplastischen Elastomeren bereits weitestgehend unterbunden werden kann.

Der elliptische Querschnitt kann beispielsweise dadurch erreicht werden, dass ein Schlauch mit ursprünglich rundem Querschnitt entsprechend verformt wird, bevor er an einem Ein- oder Auslassstutzen montiert wird. Die Verformung wird dann nicht durch die Montage bewirkt, sondern um unerwünschtes Kriechverhalten zu unterbinden, erfolgt eine Vorverformung des Schlauches auf den gewünschten elliptischen Querschnitt.

In einem anderen Ausführungsbeispiel der Okklusionssensorik ist die Sensorkomponente eine speziell geformte Messmembran mit einem Querschnitt, der wenigstens zwei gegenüber liegende Membranseiten aufweist, die jeweils nach innen eingeknickt sind, während eine Membranoberseite, welche die beiden Membranseiten miteinander verbindet, gerade ausgeformt und im Bereich der Aussparung angeordnet ist. Der Kraftsensor liegt somit an einer geraden Fläche der Messmembran an, die nicht mehr durch Eigenspannung verändert wird, so dass sich eine lineare Kraftkennlinie ergibt.

Weiterhin kann optional in einen Einlassstutzen und/oder in einem Auslassstutzen jeweils eine Ultraschallsensorik zur Erkennung von Luftblasen im jeweiligen Stutzen integriert sein, die vorzugsweise ebenfalls nicht zerstörungsfrei demontierbar ist. In einem Ausführungsbeispiel der Erfindung ist die Ultraschallsensorik so ausgeführt, dass in den Einlass- und/oder Auslassstutzen formschlüssig ein Schlauch eingesteckt ist, durch den Fluid dem Einlassstutzen zugeführt bzw. durch den Fluid aus dem Auslassstutzen abgeführt wird. Dabei sind im Bereich des Schlauches an zwei Seiten des betreffenden Einlass- und/oder Auslassstutzens Flächen zur Ein- und Auskopplung von Ultraschall vorgesehen. Diese Flächen zur Ein- und Auskopplung von Ultraschall können plan ausgeführt sein, sie können jedoch auch anders geformte Oberfläche aufweisen, die an die Form der Ultraschallsensoren angepasst sind.

Der Einlass- und/oder Auslassstutzen ist vorzugsweise so ausgeformt, dass diese Flächen in einer Ebene liegen. Ferner kann es zweckmäßig sein, dass der Einlassund/oder Auslassstutzen eine Aussparung aufweist, um eine mögliche Kurzschlussstrecke des Ultraschallwegs an dem zu untersuchenden Schlauchstück vorbei zu verhindern. In einem Ausführungsbeispiel der Erfindung liegt diese Aussparung gegenüber den Flächen zur Ein- und Auskopplung von Ultraschall, die Aussparung kann jedoch beliebig angeordnet sein. Auch mehrere Aussparungen für diesen Zweck sind möglich.

Mögliche Einsatzgebiete einer so ausgestalteten Pumpe sind u.a. medizinische Einmalartikel von Infusions- oder Dialysesystemen oder Geräte mit Einmalartikeln zur individuellen Dosierung von Medikamenten z.B. im Apothekenbereich. Hier ist das sichere Verschließen der Ein- und Auslassstutzen von entscheidender Bedeutung. Die beschriebene Anordnung kann insbesondere in ein medizinisches Infusions-Set integriert werden und kann dort das für die Förderung nötige Peristaltiksegment ersetzen. Die Erfindung ist jedoch nicht auf die Verwendung in Infusions-Sets beschränkt, sondern umfasst allgemein Vorrichtungen zur Zuführung und Dosierung eines Fluids für medizinische Zwecke, wobei die jeweilige Vorrichtung die erfindungsgemäße Kolbenpumpe umfasst.

In die Kolbenpumpe können dann neben der reinen Pumpenfunktion mechanische Vorrichtungen integriert werden, die den mechanischen Teil der geforderten Fluidsensorik bilden und eine einfache mechanische Schnittstelle zu den externen elektronischen Sensorkomponenten darstellen. Bei Ausformung mit einer Sensorkomponente in einem Stutzen kann sie somit auch das für die Okklusionssensorik wichtige Druckmesssegment ersetzen. Durch die optionale Schnittstelle zu einem Ultraschallsensor, der potentielle Luftblasen erkennen kann, wird eine weitere Voraussetzung für ein kompaktes Kassettensystem für die Pump- und Sensoreinheit geschaffen.

Die mechanischen Sensorkomponenten müssen dabei nicht als eigenständige Komponenten durch aufwändige Montage oder Assemblierungstechniken in das Pumpenmodul eingesetzt werden, sondern können integrale und nicht demontierbare Bestandteile der Pumpe bilden. Hierzu wurden intelligente Anordnungen und ein Mechanikdesign gefunden, das kostengünstige Herstellungstechniken und insbesondere Mehrkomponenten-Spritzgusstechniken berücksichtigt. Die Pumpe selbst kann als periodisch arbeitende Kolbenpumpe realisiert werden, um die Vorteile der hohen Fördergenauigkeit sowie der Fördermöglichkeit aus einem Vorratsbeutel zu vereinen.

Die beschriebene Anordnung ist jedoch nicht nur auf die Förderung von Flüssigkeiten beschränkt. Bei guter Abdichtung der Ventilscheibe ist durch das Kolbenpumpenprinzip auch die Förderung von Gasen nicht ausgeschlossen.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1a: eine schematische Darstellung des Funktionsprinzips eines Ausführungsbeispiels einer Kolbenpumpe in einer Aufsicht;
- Fig. 1b: eine Kolbenpumpe gemäß Fig. 1 in einem schematischen Querschnitt;
- Fig. 2: eine schematische Darstellung der Kolbenpumpe mit rotierender Ventilscheibe;
- Fig. 3a: das Zusammenspiel zwischen Kolbenstellung und Ventilscheibenstellung bei 0°;
- Fig. 3b: das Zusammenspiel zwischen Kolbenstellung und Ventilscheibenstellung bei 30°;
- Fig. 3c: das Zusammenspiel zwischen Kolbenstellung und Ventilscheibenstellung bei 180°;
- Fig. 3d: das Zusammenspiel zwischen Kolbenstellung und Ventilscheibenstellung bei 210°;
- Fig. 4: eine schematische Darstellung eines Ausführungsbeispiels eines Exzenterantriebs für eine erfindungsgemäße Kolbenpumpe;
- Fig. 5: das Verschließen der Ventile durch einen Exzenterantrieb gemäß Fig. 4;
- Fig. 6a: eine Aufsicht auf einen Pumpenflansch mit Sensorkomponente;
- Fig. 6b: einen Querschnitt durch einen Pumpenflansch gemäß Fig. 6a;
- Fig. 7: einen Längsschnitt durch einen Stutzen mit außen liegender Sensorkomponente;
- Fig. 8: einen Querschnitt durch einen Stutzen gemäß Fig. 7;
- Fig. 9: einen Längsschnitt durch einen Stutzen mit einem ersten Ausführungsbeispiel einer innen liegenden Sensorkomponente;
- Fig. 10: einen ersten Querschnitt durch einen Stutzen gemäß Fig. 9;
- Fig. 11: einen zweiten Querschnitt durch einen Stutzen gemäß Fig. 9;
- Fig. 12: einen Querschnitt durch einen Stutzen mit einem zweiten Ausführungsbeispiel einer innen liegenden Sensorkomponente;
- Fig. 13: einen Längsschnitt durch einen Stutzen mit innen liegender Sensorkomponente und Ultraschallsensorik;
- Fig. 14: einen Querschnitt durch einen Stutzen mit Ultraschallsensorik gemäß Fig. 13.

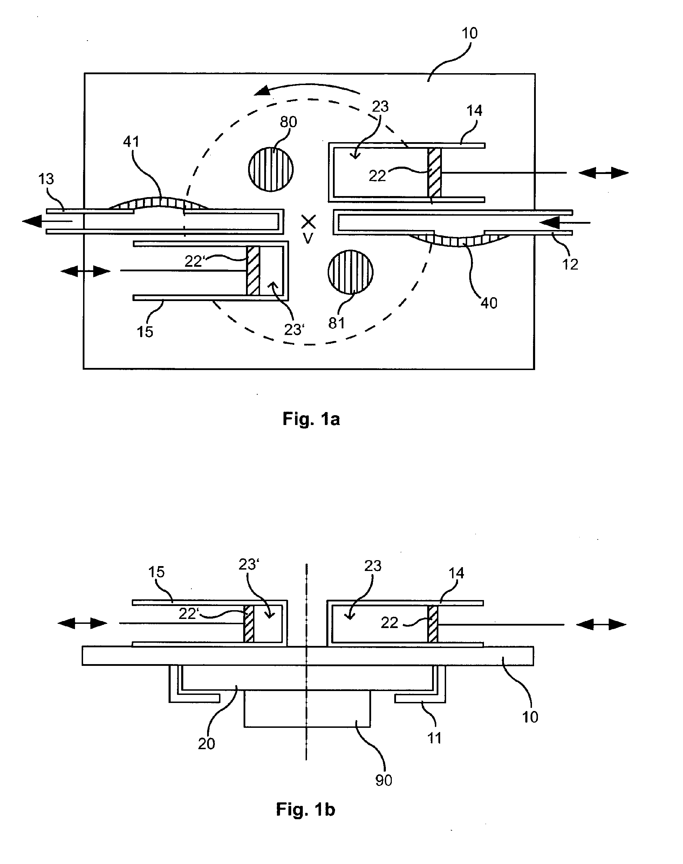

Fig. 1a zeigt eine schematische Darstellung eines Ausführungsbeispiels einer Kolbenpumpe in einer Aufsicht, was ergänzt wird durch einen entsprechenden Querschnitt in Fig. 1 b. Dabei sind auf einem ebenen Pumpenflansch 10 ein oder mehrere Zylinder 14, 15 mit je einem hin und her beweglichen Kolben 22, 22' befestigt. Weitere auf dem Flansch 10 befestigte Komponenten umfassen einen Einlassstutzen 12 und einen Auslassstutzen 13. Das zu fördernde Fluid strömt dann beispielsweise aus einem Vorratsbehälter und Schlauchsystem in den Einlassstutzen 12 ein, während es aus dem Auslassstutzen 13 herausströmt und über ein weiteres Schlauchsystem einem Patienten zugeführt wird.

Ferner ist auf dem Pumpenflansch 10, der auch als Montageflansch bezeichnet werden kann, mindestens ein Sensor 40, 41, 80, 81 vorgesehen, welcher der Erkennung einer Okklusion dient. Der Sensor für die Okklusionserkennung kann dabei entweder auf dem Flansch 10 selbst und/oder in die zu- oder abführenden Pumpenstutzen 12, 13 integriert werden. Die auf dem Flansch 10 mit Zuführungs- oder Auslasskanälen verbundenen Sensoren sind in dem Ausführungsbeispiel der Fig. 1a mit 80 und 81 gekennzeichnet, wohingegen die direkt in die Einlass- oder Auslassstutzen 12, 13 integrierten Sensoren mit 40 und 41 gekennzeichnet sind.

Die Ventilfunktion wird durch eine zentral angeordnete rotierende Ventilscheibe 20 realisiert, die sich unterhalb des Flansches 10 befindet und beispielsweise über eine Flanschhalterung 11 am Flansch 10 gelagert sein kann. Diese Ventilscheibe 20 ist über diverse Öffnungen im Flansch 10 mit den Zylindern 14, 15 bzw. den Pumpenstutzen 12, 13 verbunden. Die Ventilscheibe 20 rotiert dabei im Förderbetrieb der Pumpe stetig um ihren Drehpunkt in eine Richtung, die in Fig. 1a durch einen gebogenen Pfeil dargestellt ist, wobei der Antrieb unterhalb der Ventilscheibe 20 in Fig. 1b schematisch durch die Bezugsziffer 90 gekennzeichnet ist.

Kennzeichnend für diese Art der Pumpe ist, dass die Kolbenbewegung mechanisch aus der Drehbewegung abgeleitet wird. Innerhalb eines bestimmten Winkelzeitraums stehen die Kolben 22, 22' dabei jedoch still, um eine druckstoßfreie Ventilumschaltung zu ermöglichen. Bei dieser Umschaltung wechselt die Kolbenfunktion vom Saugbetrieb in den Förderbetrieb und umgekehrt. Eine Änderung der Rotationsrichtung der Ventilscheibe 20 kann ebenfalls zu einem Wechsel der Kolbenfunktion führen. Andererseits kann die rückwärtige Rotation auch für andere mechanische Zwecke genutzt werden.

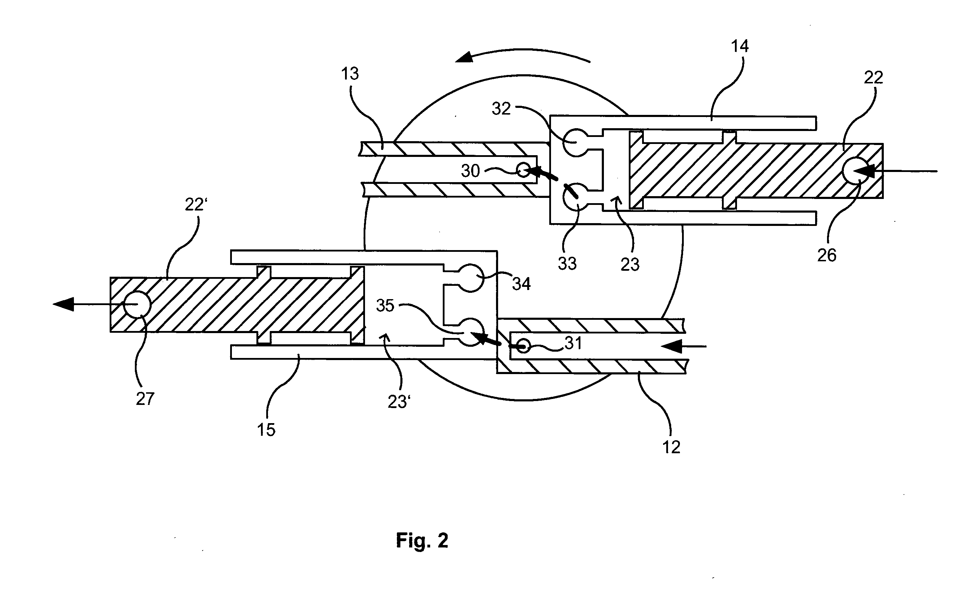

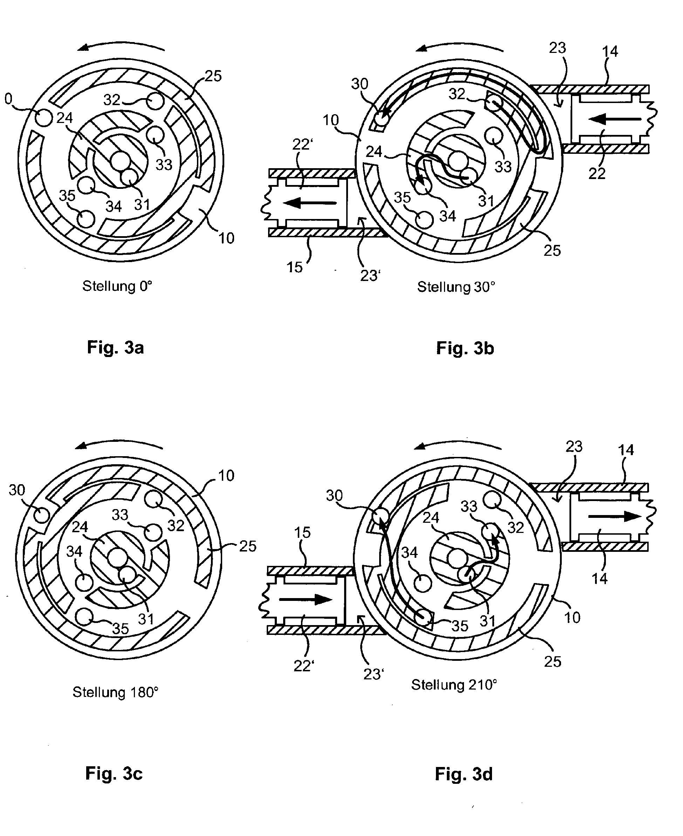

Das Zusammenspiel zwischen Ventilscheibenstellung und Kolbenfunktion ist in den Figuren 2 und 3a bis 3c dargestellt. Die Verbindung zwischen Ventilscheibe 20 und Zylindern 14, 15 erfolgt über mehrere Öffnungen, die beispielhaft durch die Öffnungen 32, 33 und 34, 35 und dargestellt sind und in einem Zylinder jeweils einen Eingang und einen Ausgang bilden. In jedem Stutzen 12, 13 sind ebenfalls jeweils Durchlässe 30 und 31 vorgesehen, welche die Stutzen mit der Ventilscheibe 20 und bei entsprechender Stellung der Ventilscheibe 20 auch mit den Zylindern 14, 15 verbinden.

Die Kolben 22, 22' stehen über einen äußeren Verbindungspunkt 26, 27 in Verbindung zu dem Antrieb 90 und werden so abwechselnd horizontal nach links und rechts bewegt. Falls mehrere Kolben 22, 22' zum Einsatz kommen, ist die Bewegungsrichtung beider Kolben 22, 22' vorzugsweise gleich.

Im Falle der in Fig. 2 dargestellten Kolbenbewegung saugt der Zylinder 15 über die Einlassöffnung 35 Fluid aus dem Einlassstutzen 12 an. Das Fluid gelangt dabei vom Einlassstutzen 12 über die Verbindungsöffnung 31 zur Ventilscheibe 20 und erreicht von dort über die Einlassöffnung 35 den Zylinder 15. Innerhalb der Ventilscheibe 20 sind dazu flanschseitig Vertiefungen entsprechend so ausgeführt, dass sie diese Verbindung herstellen oder unterbinden können, wobei dieser Verbindungsweg in Fig. 2 mit einem gestrichelten Pfeil dargestellt ist.

Im gleichen Zeitraum fördert Zylinder 14 Fluid in Richtung des Auslassstutzens 13. Dabei nimmt das Fluid vom Zylinder 14 den Weg über die Auslassöffnung 33 in die Ventilscheibe 20. Innerhalb dieser gelangt sie über einen Kanal innerhalb der Ventilscheibe 20 durch die Verbindungsöffnung 30 in den Auslassstutzen 13. Auch dieser Verbindungsweg ist in Fig. 2 durch einen gestrichelten Pfeil dargestellt. Nach einer halben Umdrehung der Ventilscheibe 20 übernimmt Zylinder 15 die Förderung der im vorangegangenen Zeitraum angesaugten Flüssigkeit über die Auslassöffnung 34 und die Öffnung 30 in den Auslassstutzen 13. Im gleichen Zeitraum saugt Zylinder 14 über dessen Einlassöffnung 32 Fluid aus dem Einlassstutzen 12 an. Hierfür muss die Ventilscheibe 20 entsprechend ausgeführt sein, um in verschiedenen Ventilscheibenstellungen unterschiedliche Verbindungswege zwischen Stutzen und Zylindern herzustellen.

Die prinzipielle Funktion der Ventilscheibe 20 wird dabei beispielsweise durch vertiefte Kanäle 24 und 25 realisiert, die auf der Flanschseite der Ventilscheibe 20 ausgebildet sind (Figuren 3a bis 3d). Die Vertiefungen 24, 25 können dabei durch kreisförmige, gebogene oder gerade vertiefte Kanäle gebildet sein. Die Dichtfunktion zwischen Ventilscheibe 20 und Flansch 10 kann unterschiedlich realisiert werden, wobei bei einer entsprechenden Abdichtung neben Flüssigkeiten auch Gase gefördert werden könnten. Durch Rotation der Ventilscheibe 20 gelangen die Kanäle 24, 25 dann unter die Zylindereinlass- oder -auslassöffnungen 32, 33, 34 und 35 und stellen eine Verbindung zu den Durchlässen 30, 31 des Einlassstutzens 12 bzw. des Auslassstutzens 13 her.

Befindet sich die Ventilscheibe 20 wie in Fig. 3a in ihrer Initialposition von 0°, ist der Kanal 25 weder mit einer Öffnung 30, 31 in einem Stutzen 12, 13, noch mit den Öffnungen 32, 33, 34, 35 zu den Zylindern 14, 15 verbunden, wodurch der Fluidstrom durch den Auslassstutzen 13 vollständig unterbunden ist. Der zweite Kanal 24 ist zwar über die Öffnung 31 mit dem Einlassstutzen 12 verbunden, eine Verbindung zu den Zylinderöffnungen 32, 33, 34, 35 ist jedoch nicht vorhanden. Dadurch ist der Fluideinlass durch den Einlassstutzen 12 ebenfalls vollständig unterbunden.

Ab einer bestimmten Winkelstellung der Ventilscheibe 20, die durch geometrische Randbedingungen gegeben ist, ist der Fluideinlass 12 zum dann saugenden Kolben 22' hin geöffnet. Gleiches gilt für den Fluidauslass 13, der mit dem ausstoßenden Kolben 22 verbunden ist. Dies ist für eine Winkelposition mit 30° in Fig. 3b dargestellt. In einer realen Ausführung kann diese Winkelposition jedoch deutlich kleiner sein und der relativ große Wert von 30° wurde lediglich aus Gründen einer übersichtlichen Darstellung gewählt. Im Detail verläuft der Einlassfluidweg in dieser Stellung vom Einlassstutzen 12 über die Öffnung 31 zum Kanal 24 der Ventilscheibe 20. Dieser Kanal 24 ist über die Öffnung 34 mit dem saugenden Kolben 22' verbunden. Der ausstoßende Kolben 22 fördert das Fluid über seine Öffnung 32 in den Kanal 25 und von dort über die Öffnung 30 in den Auslassstutzen 13.

Unterbrochen wird diese Förderrichtung wieder ab einer weiteren bestimmten Winkelposition und die Auslassöffnung 30 in den Auslassstutzen 13 ist, wie für die Winkelposition 180° in Fig. 3c gezeigt, wieder vom Kanal 25 getrennt. Kanal 24 ist ebenfalls von den Zylinderöffnungen 32, 33, 34, 35 getrennt und es ergibt sich die gleiche Verschluss-Situation wie in der Winkelstellung 0°.

Eine weitere Drehung der Ventilscheibe führt zu einer Situation, wie sie für eine Winkelposition von 210° in Fig. 3d dargestellt ist. Zylinder 15 ist nun über die Zylinderöffnung 35 mit dem Kanal 25 und von dort über die Öffnung 30 mit dem Auslassstutzen 13 verbunden, so dass Zylinder 15 vom saugenden zum ausstoßenden Zylinder geworden ist. Zylinder 14 hingegen wechselt seine Funktion von ausstoßenden zum ansaugenden Zylinder über einen Fluidweg, der vom Einlassstutzen 12 über den Durchlass 31 und die Öffnung 33 zum Zylinder 14 führt.

Eine weitere Drehung der Ventilscheibe 20 verschließt ab einer bestimmten Position wieder den Einlassstutzen 12 und den Auslassstutzen 13, wie es beispielhaft bereits für die Winkelposition von Stellung 0° in Fig. 3a dargestellt war.

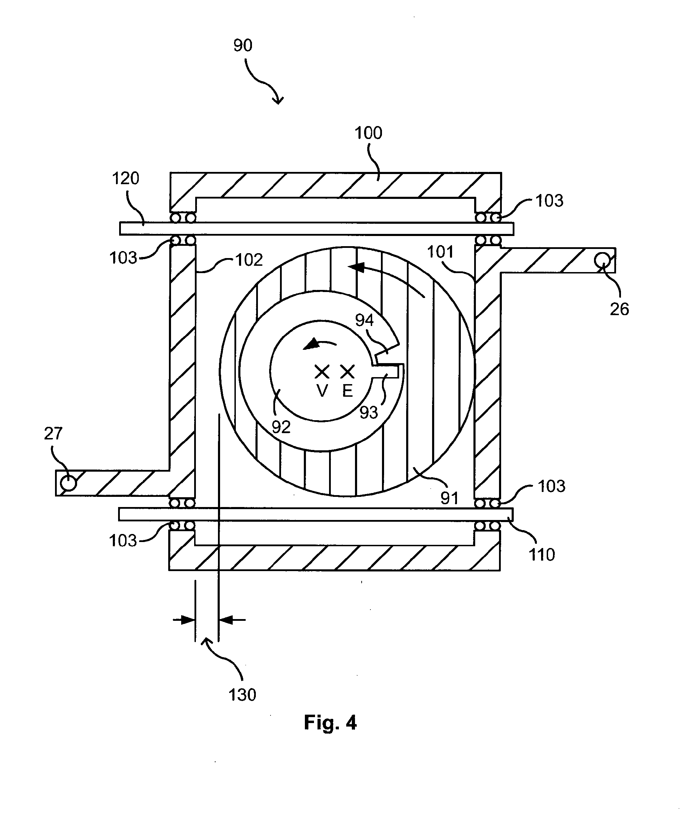

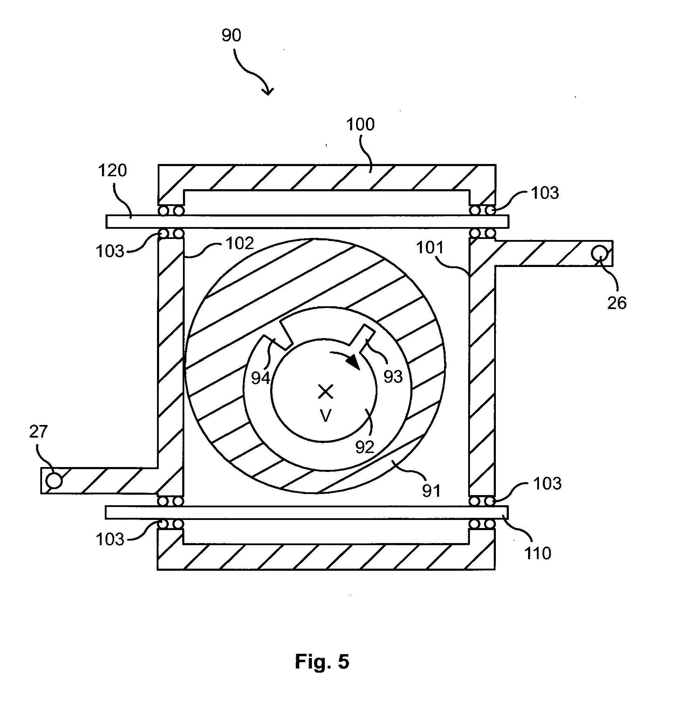

Das aus dem Ventilscheibenantrieb abgeleitete Kolbenantriebskonzept kann über unterschiedliche mechanische Konzepte realisiert werden. Dabei muss das Antriebskonzept neben der eigentlichen Kolbenbewegung einen sicheren Stillstand der Kolben 22, 22' innerhalb eines bestimmten Winkelbereichs der Ventilscheibe 20 sicherstellen, die der Funktionsumschaltung der Zylinder 14, 15 von Saug- auf Förderbetrieb und umgekehrt dient. Dies kann beispielsweise durch einen Exzenterantrieb 90 erreicht werden, wie er schematisch in den Figuren 4 und 5 dargestellt ist.

Unterhalb der Ventilscheibe 20 befindet sich bei diesem Exzenterantrieb 90 eine rotatorische Antriebseinheit 92, die direkt mit der Ventilscheibe 20 gekoppelt ist und diese um ihren mittleren Drehpunkt V dreht. Umgeben ist der rotatorische Antrieb 92 von einer ringförmigen Exzenterscheibe 91 mit einem Drehpunkt E, der von dem Drehpunkt V der Ventilscheibe 20 abweicht. Entscheidend für eine zu realisierende Free Flow-Klemmenfunktion ist, dass die Exzenterscheibe 91 nicht starr mit dem rotatorischen Antrieb 92 gekoppelt ist. Die Kopplung der Drehbewegungen wird vielmehr über eine Nase 93 bewerkstelligt, die an dem Außenumfang des rotatorischen Antriebs 92 befestigt ist. Die Kopplung der beiden Rotationen erfolgt dann in Vorwärtsdrehrichtung über einen Zapfen 94, der am Innenumfang der Exzenterscheibe 91 angebracht ist. Dreht sich der Antrieb 92 und damit die Ventilscheibe 20, wie es in Fig. 4 für eine Vorwärtsdrehrichtung durch gebogene Pfeile dargestellt ist, wird die Exzenterscheibe 91 über die Nase 93, die an dem Zapfen 94 anliegt, mitgenommen.

Die Exzenterscheibe 91 ist von einem horizontal verschiebbaren Rahmen 100 umgeben, der während ihrer Drehung an dessen Flanken 101, 102 anläuft. Über die Lager 103 ist der Rahmen 100 mit zwei horizontalen Führungsstangen 110, 120 verbunden, so dass die Rotation der Exzenterscheibe 91 zu einer horizontalen Hin- und Herbewegung des Rahmens 100 führt. Dargestellt in Fig. 4 ist dabei der rechte Umkehrpunkt des Rahmens 100. Über die Verbindungspunkte 26, 27 wird dann Kontakt zu den Kolben 22, 22' hergestellt, wobei Kolben 22 in der gezeichneten Rahmenstellung maximal ausgefahren ist, da er mit dem Verbindungspunkt 26 verbunden ist. Kolben 22' ist mit dem Verbindungspunkt 27 verbunden und vollständig eingefahren.

Aus Fig. 4 erkennt man ferner, dass der Abstand zwischen den beiden Rahmenflanken 101 und 102 größer ist als der Durchmesser der Exzenterscheibe 91. Dieser Unterschied ist durch das Spaltmaß 130 gegeben. Während der Zeit, die die Exzenterscheibe 91 benötigt, um von der Rahmenkante 101 abzulaufen und gegen die gegenüber liegende Rahmenkante 102 anzulaufen, ruhen die Kolben. Die Ventilfunktion der Ventilscheibe 20 muss innerhalb dieses Winkelbereichs vollständig umgeschaltet haben. Die Größe des Spaltmaßes 130 muss daher exakt auf die Ventilscheibengeometrie abgestimmt sein.

Wird nach Abschaltung der Förderfunktion ein zusätzlicher Verschluss der Ein- und Auslassstutzen 12, 13 gefordert, ist dies durch eine Rückwärtsbewegung der Ventilscheibe 20 möglich. Diese Situation ist in Fig. 5 dargestellt. Durch eine Rückwärtsbewegung der rotatorischen Antriebseinheit 92 löst sich die Nase 93 vom Zapfen 94 der Exzenterscheibe 91. Die Exzenterscheibe 91, der Rahmen 100 und dadurch die Kolben 22, 22' bleiben in ihrer augenblicklichen Position stehen. Durch die starre Kopplung zwischen dem rotatorischen Antrieb 92 und der Ventilscheibe 20 ist es möglich, die in den Figuren 3a und 3c mit 0° bzw. 180° gekennzeichneten Ventilscheibenpositionen anzufahren, die ein komplettes Verschließen der Stutzen 12, 13 bewirken.

Eine optional in die erfindungsgemäße Pumpe integrierbare Okklusionssensorik basiert vorzugsweise auf dem Prinzip der Druckmessung im Fluid und benutzt als mechanische Sensorschnittstelle eine Membran aus einem flexiblen Material. Diese Membran kann spritzgusstechnisch in einem Zweikomponentenverfahren hergestellt werden, wobei die Hartkomponente für das Gehäuse verwendet wird und die Weichkomponente zur Realisierung der eigentlichen Messmembran. Dabei kann ein Gehäuse als Hartkomponente wahlweise durch den Pumpenflansch ein und/oder einen Ein- und Auslassstutzen realisiert werden.

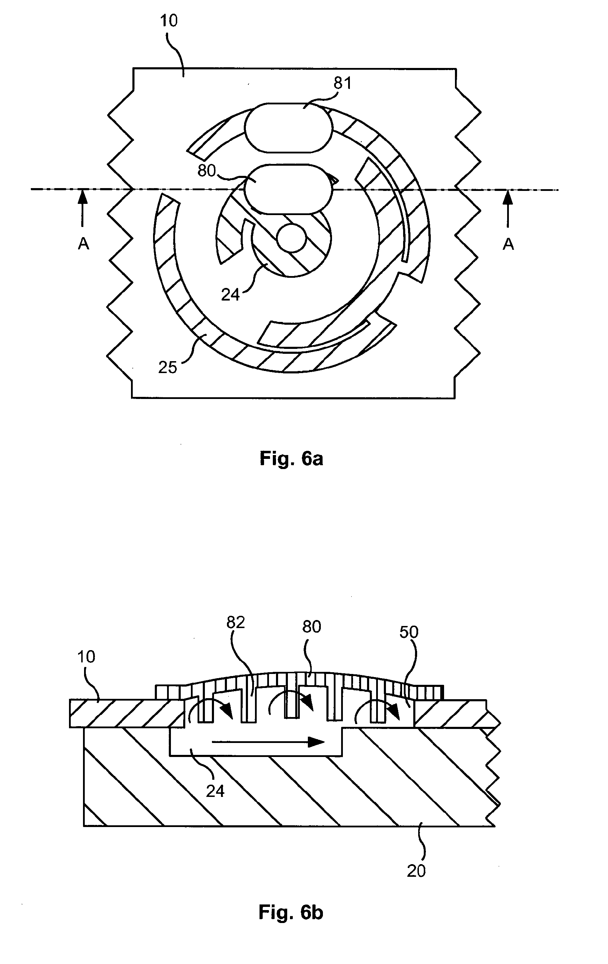

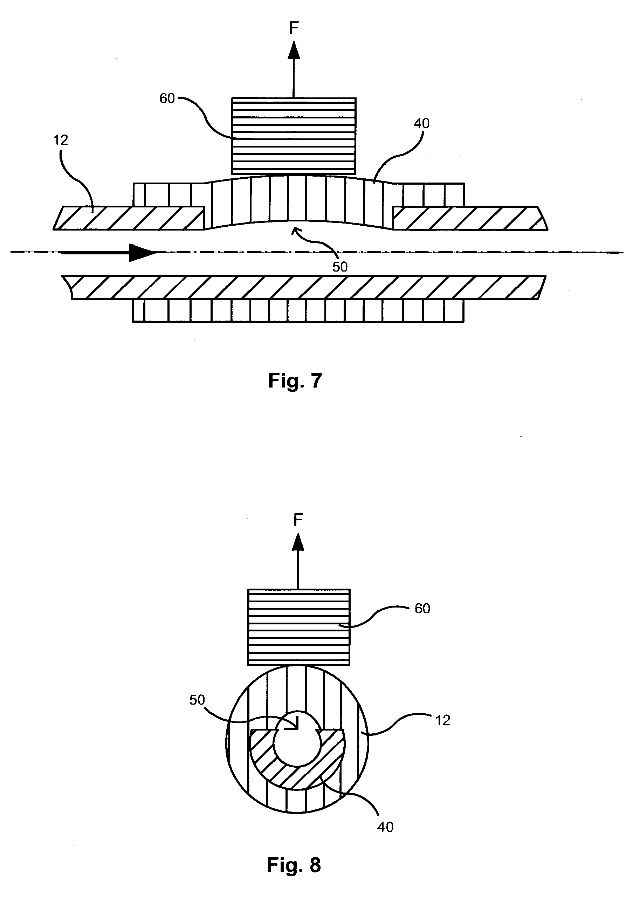

Mögliche Ausführungsformen einer solchen Okklusionssensorik sind den Figuren 6a bis 12 zu entnehmen. Figuren 6a und 6b zeigen dabei eine Ausführungsform, bei welcher eine Okklusionssensorik direkt auf dem Flansch 10 angebracht ist, während die Okklusionssensorik bei den Ausführungsformen der Figuren 7 bis 12 in einen Stutzen 12, 13 integriert sind. Beide Ausführungsformen können auch kombiniert werden.

Bei einem Lösungsvorschlag mit Okklusionssensorik direkt auf dem Flansch 10 sind beispielsweise zwei Sensorkomponenten 80, 81 in Form von Membranen auf der Oberseite des Flansches 10 aufgebracht. Dazu ist im Flansch 10 jeweils eine Aussparung 50 vorgesehen, welche durch eine jeweilige Sensorkomponente dicht abgedeckt wird. Um eine größere Fläche und damit eine erhöhte Druckempfindlichkeit bereitstellen zu können, ist die jeweilige Membran vorzugsweise deutlich breiter als die Breite der auf der Ventilscheibe 20 untergebrachten Vertiefungen 24, 25.

In der Fig. 6b ist die Hauptströmung durch einen horizontalen Pfeil nach rechts dargestellt. Möglicherweise kann das Volumen unterhalb der Membranen 80, 81 durch diese Hauptströmung jedoch nicht vollständig entlüftet wird. Die Funktion der Okklusionssensorik ist jedoch auch für diesen Fall vollständig gegeben. Durch eine zusätzliche Flüssigkeitsmenge, die benötigt wird, die Luft zu komprimieren, verzögert sich die Ansprechzeit bei einem nicht vollständig entlüfteten Volumen unterhalb der Membranen 80, 81 etwas. Wird die Membranunterseite mit einem einfach oder doppelt spiralförmigen Labyrinth 82 versehen, wird bei entsprechender Ausgestaltung der Kapillareffekt eine zusätzliche Anfangsströmung induzieren, die eine weitest gehende Entlüftung bewirken kann. Diese Anfangsströmung ist in Fig. 6b durch mehrere gebogene Pfeile im Uhrzeigersinn dargestellt.

Die von den Membranen der Sensorkomponenten 80, 81 ausgehende Kraft wird auf einen externen Kraftsensor gegeben, um eine Okklusion zu erkennen. Da eine Okklusion in der Pumpenzuleitung 12 beim Ansaugen der Pumpe einen Unterdruck zur Folge hat, müssen dort eingesetzte Membranen durch ihr Design bereits eine Wölbung aufweisen, die sich durch den Unterdruck verringert.

Eine alternative Ausführung der Okklusionssensorik an den Ein- und Auslassstutzen 12 und 13 ist in den Figuren 7 bis 12 gezeigt, wobei in den Figuren beispielhaft der betreffende Bereich in einem Einlassstutzen 12 mit einer Sensorkomponente 40 dargestellt ist.

Der in Fig. 7 dargestellte Längsschnitt durch einen Stutzen 12 zeigt eine außen liegende Sensorkomponente 40, welche den Stutzen 12 im Bereich einer Aussparung 50 formschlüssig umgibt. Hierbei wird eine dichte Verbindung zwischen dem Stutzen 12 und der schlauchförmigen Sensorkomponente 40 erreicht. Die Sensorkomponente 40 kann dabei an ihrer Innenseite so ausgeformt sein, dass sie sich teilweise in die Aussparung 50 einfügt, wie es in Fig. 7 dargestellt ist. Die Okklusionssensorik kann dabei vorteilhafterweise in einem spritzgusstechnischen Zweikomponentenprozess gefertigt werden, wobei die Sensorkomponente 40 als zweiter Prozessschritt nach der Herstellung eines rohrförmigen Stutzens 12 durch eine Hartkomponente aufgebracht wird. Als Material für die Hartkomponente kann ein Hartkunststoff gewählt werden, während die Sensorkomponente aus einem elastischen und druckempfindlichen Material wie einem Elastomer gefertigt wird.

Die Aussparung 50 kann einen beliebigen Querschnitt haben, wobei sich runde Querschnitte für eine gleichmäßige Kraftverteilung als vorteilhaft erwiesen haben. Ferner sollte die Größe der Aussparung 50 geeignet gewählt werden. In Fig. 8 ist beispielsweise ein Querschnitt durch die Mitte des Längsschnitts aus Fig. 7 gezeigt, bei dem die Aussparung 50 sehr tief gewählt wurde und annähernd bis zur Mittellinie des Stutzens 12 reicht.

Durch die Aussparung 50 kann dann ein Kraftsensor 60 reichen, um in diesem Bereich Kontakt mit der Außenseite der Sensorkomponente 40 herzustellen und die Verformung der Membran 20 mechanisch abzugreifen. Dies kann beispielsweise über einen Stempel 60 erfolgen, der an der Sensorkomponente 40 anliegt. Erhöht sich der Innendruck im Stutzen 12 aufgrund einer Okklusion, wölbt sich die Sensorkomponente 40 weiter nach außen, was von dem Stempel 60 detektiert werden kann. Reduziert sich der Druck im Stutzen 12 aufgrund einer Okklusion, verringert sich die Wölbung der Sensorkomponente 41, was ebenfalls durch den Stempel 60 detektiert werden kann.

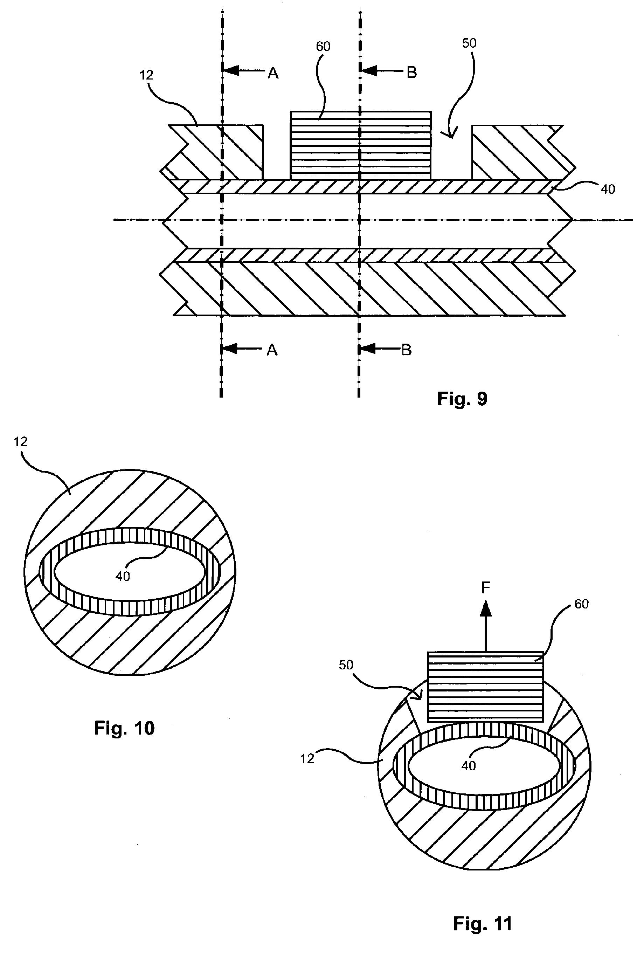

Fig. 9 zeigt ein zweites Ausführungsbeispiel der Erfindung, bei dem eine schlauchförmige Sensorkomponente 40 innerhalb eines Stutzens 12 angebracht ist und somit von innen eine Aussparung 50 dicht abdeckt. Diese Okklusionssensorik kann ebenfalls in einem spritzgusstechnischen Zweikomponentenprozess in Form eines durchgehenden Innenschlauchs als Weichkomponente hergestellt werden, während der zugehörige Einlass- oder Auslassstutzen als darüber liegendes Stützrohr bzw. Stützskelett als Hartkomponente integral und nicht zerlegbar gefertigt wird. Dabei kann die Innenfläche des Stutzens 12 auch so ausgebildet sein, dass sie den Schlauch 40 in seiner Position hält und an einem axialen Verrutschen hindert (nicht dargestellt).

Fig. 10 zeigt einen ersten Querschnitt durch einen solchen Stutzen entlang der Linie A-A, wodurch ersichtlich wird, dass die Sensorkomponente 40 einen elliptischen Querschnitt hat. Die Innenwandung des Stutzens 12 ist entsprechend ausgeformt, um die Sensorkomponente 40 formschlüssig aufnehmen zu können. Ein zweiter Querschnitt entlang der Linie B-B ist in Fig. 11 dargestellt und zeigt auch den Stempel 60, der durch die Aussparung 50 hindurch die Außenfläche der Sensorkomponente kontaktiert.

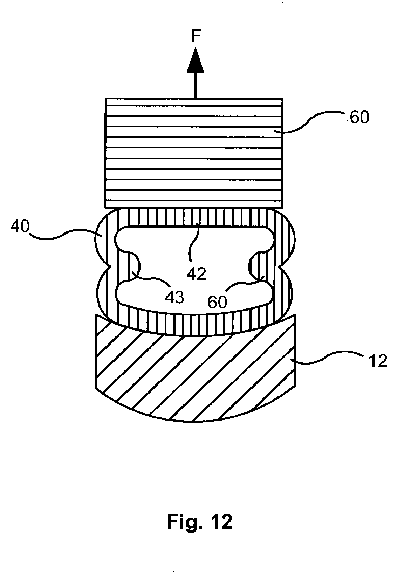

Um Eigenspannungen der Sensorkomponente 40 weitestgehend zu verhindern, kann diese auch als speziell geformte Messmembran ausgebildet sein, wie sie beispielsweise in Fig. 12 dargestellt ist. Hierbei weist die Messmembran 40 zwei gegenüber liegende Membranseiten 43 und 44 auf, die nach innen eingeknickt sind. Die Membranoberseite 42, welche die beiden Membranseiten 43, 44 verbindet, ist gerade ausgeführt und steht in Kontakt mit dem Stempel 60. Die Membranoberseite 42 verändert sich so nicht mehr durch die Eigenspannung, was eine lineare Kraftkennlinie Kraft = Innendruck x Membranfläche ergibt.

Die Innenfläche des Stutzens 12 kann dann entsprechend ausgeführt sein, so dass die Messmembran 40 formschlüssig an diesem anliegt und sich bei einer Druckerhöhung nicht in unerwünschte Richtungen z.B. zur Seite hin ausdehnt. Diese spezielle Ausformung des Stutzens 12 kann auch nur im Bereich der Okklusionssensorik gegeben sein, wodurch sich aufwändige Formen innerhalb des gesamten Stutzens vermeiden lassen.

Der Querschnitt der Sensorkomponente 40 ist somit individuell gestaltet und enthält wenigstens eine der folgenden funktionalen Komponenten:
□ Eine gerade oder einer Geraden ähnliche Linie, welche die Geometrie der für die Messzwecke benötigte Membran bestimmt
□ Eine gerade oder gebogene Linie gegenüber der Membran, die eine Stützfunktion der Weichkomponente gegenüber der rohr- oder skelettförmigen Hartkomponente wahrnimmt.
□ Eine Geometrie zur Realisierung einer federnden Funktion an den beiden Seiten der Weichkomponente, damit eine Vorspannung aufgebaut werden kann, die zur Messung von Drücken unterhalb des atmosphärischen Umgebungsdrucks notwendig ist. Darüber hinaus ist die federnde Funktion notwendig, damit sich die Membran bei einem steigenden Innendruck von ihrer gegenüberliegenden Stützfläche entfernen kann.

Die Hartkomponente, die den Stempel 60 umgibt, weist vorzugsweise ferner eine ebene Fläche auf, die etwas unterhalb der Stempeloberkante liegt. Diese Fläche dient als Anschlagsfläche, wenn der Stempel gegen eine andere Fläche gedrückt wird. Der Stempel kann dann nur um den Betrag seines Überstandes eingedrückt werden, wodurch eine konstante Vorspannung für die Drucksensorik erzeugt wird.

Bei den in den Figuren 7 bis 12 dargestellten Ausführungsbeispielen befindet sich die Aussparung 50 und somit der Stempel 60 im Stutzen 12 stets oben, es können jedoch auch andere Anordnungen gewählt werden.

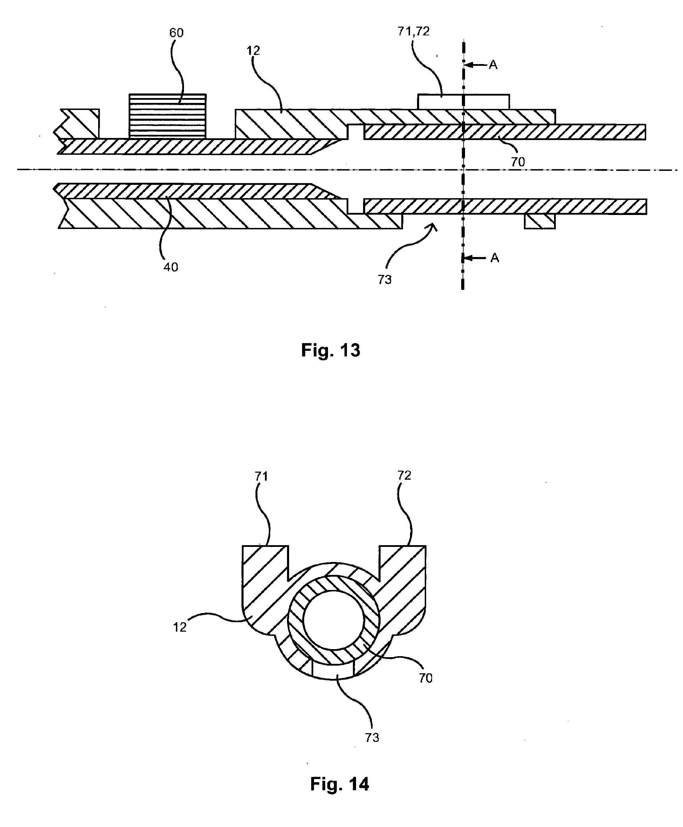

In Fig. 13 ist zusammen mit einer innen liegenden Sensorkomponente 40 eine optionale Ultraschallsensorik für die erfindungsgemäße Kolbenpumpe gezeigt, die das zusätzliche manuelle Einführen eines Schlauchs in spezielle Halterungen überflüssig macht. Mittels dieser Ultraschallsensorik können Luftblasen im Infusionsschlauchsystem erkannt werden, wobei auch diese Ultraschallsensorik im Einlass- und/oder Auslassstutzen 12, 13 angebracht werden kann. In Fig. 13 ist die Ultraschallsensorik beispielhaft im Einlassstutzen 12 dargestellt. Hierzu ist dieser Stutzen 12 an seinem Ende innen entsprechend aufgeweitet, so dass dort ein flexibler Schlauch 70 eingeführt und beispielsweise durch Kleben fixiert werden kann. Die Ein- und Auskopplung des Ultraschalls erfolgt an zwei gegenüberliegenden Flächen 71, 72, die vorzugsweise plan ausgeführt sein können, wie es in Fig. 14 im Querschnitt entlang der Linie A-A gezeigt ist. Die beiden Flächen 71, 72 liegen dabei in einer Ebene. Gegenüber den planen Flächen 71, 72 befindet sich eine Aussparung 73 im Einlassstutzen 12. Es sind jedoch auch andere formschlüssige Verbindungen wie z.B. über einen Konus denkbar.

Wie die Okklusionssensorik bilden die mechanischen Komponenten für die Luftblasenerkennung vorzugsweise ebenfalls einen integralen Bestandteil der rohrförmigen Stutzen und können nicht zerstörungsfrei demontiert werden. Vergleichbare Anpassungen der Pumpvorrichtung zur Unterstützung der Sensorik sind ebenfalls möglich, um beispielsweise alternative optische Luftblasenerkennungsmethoden oder die Bildung von definierten Schnittstellen für eine Temperaturmessung zu ermöglichen.

Die Ein- und Auskoppelflächen für den Ultraschall sowie die Anschlagsfläche für die Okklusionssensorik bilden vorzugsweise eine Ebene, wodurch die Schnittstelle zu den zugehörigen elektronischen Sensoren ebenfalls eine Ebene bildet, die sich z.B. in einem medizinischen Gerät befinden kann. Durch diese Maßnahme lassen sich Anforderungen an eine gute und einfache Reinigungsmöglichkeit gut umsetzen.

### Bezugszeichenliste

- 10: Flansch, Pumpenflansch
- 11: Flanschhalterung
- 12: Stutzen, Einlassstutzen
- 13: Stutzen, Auslassstutzen
- 14,15: Zylinder
- 20: Ventilscheibe
- 22,22': Kolben
- 23,23': Kammer
- 24,25: Vertiefung, Kanal
- 26,27: Verbindungspunkt Antrieb-Kolben
- 30,31: Durchlass
- 32,33,34,35: Zylinderöffnung

- 40,41: Sensorkomponente an Stutzen, Messmembran
- 42: Membranoberseite
- 43,44: Membranseite
- 50: Aussparung für Okklusionssensorik
- 60: Kraftsensor, Stempel
- 70: Schlauchstück
- 71,72: Ein- und Auskopplungsfläche
- 73: Aussparung für Ultraschallsensorik
- 80,81: Sensorkomponente an Pumpenflansch, Messmembran
- 82: Labyrinth

- 90: Antrieb, Exzenterantrieb
- 91: Exzenterscheibe
- 92: Antriebseinheit
- 93: Nase
- 94: Zapfen
- 100: Rahmen
- 101,102: Rahmenflanke
- 103: Lager
- 110,120: Führungsstange
- 130: Spalt

- V: Drehpunkt Ventilscheibe
- E: Drehpunkt Exzenterscheibe

## Patentansprüche

1. Kolbenpumpe zur Förderung eines Fluids, umfassend wenigstens zwei Zylinder (14;15) mit jeweils einem Kolben (22;22'), der mittels eines Antriebs (90) innerhalb des zugehörigen Zylinders (14;15) entlang der Längsachse des Zylinders (14;15) bewegbar ist, wobei die Zylinder (14;15) auf einem gemeinsamen Pumpenflansch (10) angebracht sind, und sich in jedem Zylinder (14;15) eine Kammer (23;23') mit veränderlichem Volumen bildet, wenn der zugehörige Kolben (22;22') im Zylinder (14;15) bewegt wird,
**dadurch gekennzeichnet, dass** sich der Pumpenflansch (10) entlang der Bewegungsrichtung der Kolben (22;22') erstreckt und auf dem Pumpenflansch (10) wenigstens ein Einlassstutzen (12) und ein Auslassstutzen (13) angebracht sind, deren Längsachsen entlang dem Pumpenflansch (10) verlaufen, dass auf der den Zylindern (14;15) abgewandten Seite des Pumpenflansches (10) eine zentrale Ventilscheibe (20) angebracht ist, die an dem Pumpenflansch (10) anliegt und sich bei Förderbetrieb der Kolbenpumpe stetig dreht, wobei die Drehachse der Ventilscheibe (20) quer zum Pumpenflansch (10) verläuft, dass im Bereich der Einlass- und Auslassstutzen (12;13) jeweils Durchlässe (30;31) und im Bereich der Zylinder (14;15) jeweils Zylinderöffnungen (32;33;34;35) in den Pumpenflansch (10) eingebracht sind, wobei die Ventilscheibe (20) flanschseitig wenigstens zwei Vertiefungen (24;25) aufweist, von denen eine erste Vertiefung (25) bei Drehung der Ventilscheibe (20) in eine erste Winkelposition mit einer Zylinderöffnung (32) eines ersten Zylinders (14) und einem Durchlass (30) des Auslassstutzens (13) in Deckung ist, während die zweite Vertiefung (24) in dieser ersten Winkelposition mit dem Durchlass (31) des Einlassstutzens (12) und einer Zylinderöffnung (34) in dem zweiten Zylinder (15) in Deckung ist, und dass die erste Vertiefung (25) bei Drehung der Ventilscheibe (20) in eine zweite Winkelposition mit dem Durchlass (30) im Auslassstutzen (13) und einer Zylinderöffnung (35) im zweiten Zylinder (15) in Deckung ist, während die zweite Vertiefung (24) in dieser zweiten Winkelposition mit dem Durchlass (31) im Einlassstutzen (12) und einer Zylinderöffnung (33) im ersten Zylinder (14) in Deckung ist.

2. Kolbenpumpe nach Anspruch 1,
**dadurch gekennzeichnet, dass** die Vertiefungen (24;25) der Ventilscheibe (20) bei Drehung der Ventilscheibe (20) in wenigstens eine dritte Winkelposition mit keiner Zylinderöffnung (32;33,34;35) in Deckung sind.

3. Kolbenpumpe nach Anspruch 2,
**dadurch gekennzeichnet, dass** die wenigstens eine dritte Winkelposition zwischen den beiden Winkelpositionen des Anspruchs 1 liegt.

4. Kolbenpumpe nach einem oder mehreren der Ansprüche 1 bis 3,
**dadurch gekennzeichnet, dass** die Längsachsen der Zylinder (14;15) und/oder die Längsachsen der Einlass- und Auslassstutzen (12,13) in die gleiche Richtung verlaufen.

5. Kolbenpumpe nach einem oder mehreren der Ansprüche 1 bis 4,
**dadurch gekennzeichnet, dass** der Antrieb (90) sowohl die Bewegung der Kolben (22;22') als auch die Drehung der Ventilscheibe (20) bewirkt.

6. Kolbenpumpe nach einem oder mehreren der Ansprüche 1 bis 5,
**dadurch gekennzeichnet, dass** der Antrieb (90) ein Exzenterantrieb mit einer Exzenterscheibe (91) und einem die Exzenterscheibe (91) umgebenden Rahmen (100) ist, wobei der Drehpunkt (V) der Ventilscheibe (20) von dem Drehpunkt (E) der Exzenterscheibe (91) abweicht, und dass eine rotatorische Antriebseinheit (92) bei Förderbetrieb der Kolbenpumpe die Drehung der Ventilscheibe (20) bewirkt, wobei die Antriebseinheit (92) so an die Exzenterscheibe (91) gekoppelt ist, dass sich die Exzenterscheibe (91) mit der Ventilscheibe (20) dreht und an zwei gegenüber liegenden Rahmenflanken (101;102) an den Rahmen (100) anläuft, wodurch sich der Rahmen (100) in Richtung der beiden Rahmenflanken (101;102) hin- und her bewegt, und dass die Kolben (22,22') jeweils so an den Rahmen (100) gekoppelt sind, dass die Hin- und Herbewegung des Rahmens (100) auf die Kolben (22;22') übertragbar ist.

7. Kolbenpumpe nach Anspruch 6,
**dadurch gekennzeichnet, dass** die Exzenterscheibe (91) lösbar an die Antriebseinheit (92) gekoppelt ist.

8. Kolbenpumpe nach Anspruch 7,
**dadurch gekennzeichnet, dass** die Exzenterscheibe (91) ringförmig ausgebildet ist und die rotatorische Antriebseinheit (92) umgibt, wobei die Antriebseinheit (92) an ihrem Außenumfang eine Nase (93) aufweist, während die Exzenterscheibe (91) an ihrem Innenumfang einen Zapfen (94) aufweist, der bei Förderbetrieb der Kolbenpumpe an der Nase (93) anliegt.

9. Kolbenpumpe nach einem oder mehreren der Ansprüche 1 bis 8,
**dadurch gekennzeichnet, dass** in den Einlassstutzen (12), den Auslassstutzen (13) und/oder den Pumpenflansch (10) jeweils wenigstens eine Okklusionssensorik integriert ist, die nicht zerstörungsfrei demontierbar ist, wobei der Einlassstutzen (12), der Auslassstutzen (13) und/oder der Pumpenflansch (10) als Gehäuse der Okklusionssensorik dienen.

10. Kolbenpumpe nach Anspruch 9,
**dadurch gekennzeichnet, dass** in dem Gehäuse wenigstens eine Aussparung (50) vorgesehen ist, die dicht durch eine Sensorkomponente (40;41) abgedeckt ist, die aus einem druckempfindlichen Material besteht, wobei das Material des Gehäuses härter ist als das der Sensorkomponente (40;41), und dass die Kolbenpumpe einen Kraftsensor (60) aufweist, mit dem druckinduzierte Veränderungen der Sensorkomponente (40;41) im Bereich der jeweiligen Aussparung (50) messbar sind.

11. Kolbenpumpe nach Anspruch 10,
**dadurch gekennzeichnet, dass** die Verbindung zwischen der Sensorkomponente (40) und einem Gehäuse eine Spritzgussverbindung ist, die durch ein Zweikomponentenverfahren hergestellt wurde.

12. Kolbenpumpe nach einem oder beiden der Ansprüche 10 und 11,
**dadurch gekennzeichnet, dass** die Sensorkomponente (40;41) schlauchförmig ist und so an einem Einlass- und/oder Auslassstutzen (12;13) angebracht ist, dass sie die jeweilige Aussparung (50) von innen oder von außen dicht abdeckt.

13. Kolbenpumpe nach einem oder mehreren der Ansprüche 1 bis 12,
**dadurch gekennzeichnet, dass** in einen Einlassstutzen (12) und/oder in einen Auslassstutzen (13) jeweils eine Ultraschallsensorik zur Erkennung von Luftblasen im jeweiligen Stutzen (12;13) integriert ist, die nicht zerstörungsfrei demontierbar ist.

14. Kolbenpumpe nach Anspruch 13,
**dadurch gekennzeichnet, dass** in den Einlass- und/oder Auslassstutzen (12;13) ein Schlauch (70) formschlüssig eingesteckt ist, durch den Fluid dem Einlassstutzen (12) zugeführt oder durch den Fluid aus dem Auslassstutzen (13) abgeführt wird, wobei im Bereich des Schlauches (70) an zwei Seiten des betreffenden Einlass- und/oder Auslassstutzens (12;13) Flächen (71;72) zur Ein- und Auskopplung von Ultraschall vorgesehen sind.

15. Vorrichtung zur Zuführung und Dosierung eines Fluids für medizinische Zwecke,
**dadurch gekennzeichnet, dass** sie eine Kolbenpumpe nach einem oder mehreren der Ansprüche 1 bis 14 umfasst.

## Claims

1. Piston pump for pumping a fluid, comprising at least two cylinders (14;15) each having a piston (22;22') which is movable inside the associated cylinder (14; 15) along the longitudinal axis of the cylinder (14; 15) by means of a drive (90), wherein the cylinders (14;15) are attached to a common pump flange (10), and in each cylinder (14;15) a chamber (23;23') is formed having a volume that changes when the associated piston (22; 22') is moved in the cylinder (14; 15),
**characterized in that** the pump flange (10) extends along the direction of motion of the pistons (22; 22'), and to the pump flange (10) are attached at least one inlet port (12) and one outlet port (13) whose longitudinal axes run along the pump flange (10), that a central valve plate (20) is attached to the side of the pump flange (10) facing away from the cylinders (14;15), which valve plate (20) bears on the pump flange (10) and continuously rotates during pumping operation of the piston pump, wherein the axis of rotation of the valve plate (20) runs transversely to the pump flange (10), that respective passages (30;31) in the region of the inlet and outlet port (12;13), and respective cylinder openings (32;33;34;35) in the region of the cylinders (14;15), are introduced to the pump flange (10), wherein the valve plate (20) comprises on the flange side at least two cavities (24;25) of which a first cavity (25) coincides, upon rotation of the valve plate (20) to a first angular position, with a cylinder opening (32) of a first cylinder (14) and a passage (30) of the outlet port (13), while the second cavity (24) coincides in this first angular position with the passage (31) of the inlet port (12) and a cylinder opening (34) in the second cylinder (15), and that the first cavity (25), upon rotation of the valve plate (20) to a second angular position, coincides with the passage (30) in the outlet port (13) and a cylinder opening (35) in the second cylinder (15), while the second cavity (24) coincides in this second angular position with the passage (31) in the inlet port (12) and a cylinder opening (33) in the first cylinder (14).

2. Piston pump according to claim 1,
**characterized in that** upon rotation of the valve plate (20) to at least a third angular position, the cavities (24;25) of the valve plate (20) do not coincide with a cylinder opening (32;33,34;35).

3. Piston pump according to claim 2,
**characterized in that** the at least one third angular position lies between the two angular positions of claim 1.

4. Piston pump according to one or more of claims 1 to 3,
**characterized in that** the longitudinal axes of the cylinders (14;15) and/or the longitudinal axes of the inlet and outlet ports (12, 13) run in the same direction.

5. Piston pump according to one or more of claims 1 to 4,
**characterized in that** the drive (90) brings about the movement of the pistons (22;22') as well as the rotation of the valve plate (20).

6. Piston pump according to one or more of claims 1 to 5,
**characterized in that** the drive (90) is an eccentric drive having an eccentric disc (91) and a frame (100) surrounding the eccentric disc (91), wherein the rotation point (V) of the valve plate (20) deviates from the rotation point (E) of the eccentric disc (91) and that a rotational drive unit (92) brings about the rotation of the valve plate (20) during pumping operation of the piston pump, wherein the drive unit (92) is coupled to the eccentric disc (91) such that the eccentric disc (91) rotates with the valve plate (20) and makes contact on the frame at two oppositelying frame flanks (101;102), whereby the frame (100) reciprocates in the direction of the two frame flanks (101;102), and that the pistons (22,22') are each coupled to the frame (100) such that the reciprocating motion of the frame (100) can be transferred to the pistons (22;22').

7. Piston pump according to claim 6,
**characterized in that** the eccentric disc (91) is releasably coupled to the drive unit (92).

8. Piston pump according to claim 7,
**characterized in that** the eccentric disc (91) is configured to be ring-shaped, and surrounds the rotational drive unit (92), wherein the drive unit (92) comprises a nose (93) at its outer periphery while the eccentric disc (91) comprises a pin (94) at its inner periphery, which bears on the nose (93) during pumping operation of the piston pump.

9. Piston pump according to one or more of claims 1 to 8,
**characterized in that** at least one respective occlusion sensor is integrated into the inlet port (12), outlet port (13) and/or the pump flange (10) respectively, which occlusion sensor cannot be non-destructively disassembled, wherein the inlet port (12), the outlet port (13) and/or the pump flange (10) serve as a housing of the occlusion sensor.

10. Piston pump according to claim 9,
**characterized in that** in the housing at least one recess (50) is provided which is tightly covered by a sensor component (40;41) composed of a pressure sensitive material, wherein the material of the housing is harder than that of the sensor component (40;41), and that the piston pump comprises a force sensor (60) with which pressure-induced changes of the sensor component (40;41) in the region of the respective recess (50) can be measured.

11. Piston pump according to claim 10,
**characterized in that** the connection between the sensor component (40) and a housing is an injection-molded connection made by a two-component process.

12. Piston pump according to one or both of claims 10 and 11,
**characterized in that** the sensor component (40;41) is tubular and is attached to an inlet and/or outlet port (12; 13) such that it tightly covers the respective recess (50) from inside or from outside.

13. Piston pump according to one or more of claims 1 to 12,
**characterized in that** an ultrasound sensor is integrated into an inlet port (12) and/or outlet port (13) respectively, for the detection of air bubbles in the respective port (12; 13), which ultrasound sensor cannot be non-destructively disassembled.

14. Piston pump according to claim 13,
**characterized in that** a tube (70) is inserted into the inlet and/or outlet port (12; 13) with form-locking fit, through which fluid is supplied to the inlet port (12) or discharged from the outlet port (13), wherein surfaces (71; 72) for the coupling and decoupling of ultrasound are provided in the region of the tube (70) on two sides of the relevant inlet and/or outlet port (12; 13).

15. Apparatus for supplying and metering a fluid for medical purposes,
**characterized in that** it includes a piston pump according to one or more of claims 1 to 14.

## Revendications

1. Pompe à pistons destinée à transporter un fluide, comprenant au moins deux vérins (14 ; 15) ayant chacun un piston (22 ; 22') qui peut être déplacé au moyen d'un entraînement (90) à l'intérieur du vérin correspondant (14 ; 15) le long de l'axe longitudinal du vérin (14, 15), les vérins (14 ; 15) étant installés sur une bride de pompe (10) commune et une chambre (23 ; 23') ayant un volume variable se formant dans chaque vérin (14 ; 15) lorsque le piston correspondant (22 ; 22') est déplacé dans le vérin (14 ; 15),
**caractérisée en ce que** la bride de pompe (10) s'étend le long de la direction de déplacement des pistons (22 ; 22') et **en ce que** sur la bride de pompe (10) sont installées au moins une tubulure d'entrée (12) et une tubulure de sortie (13) dont les axes longitudinaux s'étendent le long de la bride de pompe (10), **en ce que** sur le côté de la bride de pompe (10) opposé aux vérins (14 ; 15) est installé un disque de soupape central (20) qui prend appui sur la bride de pompe (10) et tourne constamment lors du fonctionnement de transport de la pompe à pistons, l'axe de rotation du disque de soupape (20) s'étendant transversalement à la bride de pompe (10), **en ce que** des passages (30 ; 31) respectifs sont réalisés dans la zone des tubulures d'entrée et de sortie (12 ; 13) et des orifices de vérin (32 ; 33 ; 34 ; 35) respectifs sont réalisés dans la zone des vérins (14 ; 15) dans la bride de pompe (10), le disque de soupape (20) présentant du côté de la bride au moins deux enfoncements (24 ; 25), dont un premier enfoncement (25), lors de la rotation du disque de soupape (20) dans une première position d'angle, est en recouvrement avec un orifice de vérin (32) d'un premier vérin (14) et un passage (30) de la tubulure de sortie (13), tandis que le deuxième enfoncement (24), dans cette première position d'angle, est en recouvrement avec le passage (31) de la tubulure d'entrée (12) et un orifice de vérin (34) dans le deuxième vérin (15), et **en ce que** le premier enfoncement (25), lors de la rotation du disque de soupape dans une deuxième position d'angle, est en recouvrement avec le passage (30) dans la tubulure de sortie (13) et un orifice de vérin (35) dans le deuxième vérin (15), tandis que le deuxième enfoncement (24), dans cette deuxième position d'angle, est en recouvrement avec le passage (31) dans la tubulure d'entrée (12) et un orifice de vérin (33) dans le premier vérin (14).

2. Pompe à pistons selon la revendication 1,
**caractérisée en ce que** les enfoncements (24 ; 25) du disque de soupape (20), lors de la rotation du disque de soupape (20) dans au moins une troisième position d'angle, ne sont en recouvrement avec aucun orifice de vérin (32 ; 33 ; 34 ; 35).

3. Pompe à pistons selon la revendication 2,
**caractérisée en ce que** l'au moins une troisième position d'angle est située entre les deux positions d'angle de la revendication 1.

4. Pompe à pistons selon l'une ou plusieurs des revendications 1 à 3,
**caractérisée en ce que** les axes longitudinaux des vérins (14 ; 15) et/ou les axes longitudinaux des tubulures d'entrée et de sortie (12 ; 13) s'étendent dans la même direction.

5. Pompe à pistons selon l'une ou plusieurs des revendications 1 à 4,
**caractérisée en ce que** l'entraînement (90) produit à la fois le déplacement des pistons (22 ; 22') et la rotation du disque de soupape (20).

6. Pompe à pistons selon l'une ou plusieurs des revendications 1 à 5,
**caractérisée en ce que** l'entraînement (90) est un entraînement à excentrique comprenant un disque d'excentrique (91) et un châssis (100) entourant le disque d'excentrique (91), le point de rotation (V) du disque de soupape (20) différant du point de rotation (E) du disque d'excentrique (91), **en ce qu'**une unité d'entraînement rotative (92) produit la rotation du disque de soupape (20) lors du fonctionnement de transport de la pompe à pistons, l'unité d'entraînement (92) étant couplée au disque d'excentrique (91) de telle manière que le disque d'excentrique (91) tourne avec le disque de soupape (20) et s'approche du châssis (100) sur deux flancs du châssis situés en opposition (101 ; 102), ce par quoi le châssis (100) est déplacé dand un mouvement de va-et-vient dans la direction des deux flancs du châssis (101 ; 102), et **en ce que** les pistons (22 ; 22') sont chacun couplés au châssis (100) de telle manière que le déplacement de va-et-vient du châssis (100) peut être transmis aux pistons (22 ; 22').

7. Pompe à pistons selon la revendication 6,
**caractérisée en ce que** le disque d'excentrique (91) est couplé à l'unité d'entraînement (92) de façon détachable.

8. Pompe à pistons selon la revendication 7,
**caractérisée en ce que** le disque d'excentrique (91) est réalisé dans une forme annulaire et entoure l'unité d'entraînement rotative (92), l'unité d'entraînement (92) présentant un bec (93) sur son pourtour extérieur, tandis que le disque d'excentrique (91) présente sur son pourtour intérieur un tenon (94) qui prend appui sur le bec (93) lors du fonctionnement de transport de la pompe à pistons.

9. Pompe à pistons selon l'une ou plusieurs des revendications 1 à 8,
**caractérisée en ce qu'**un dispositif de détection d'occlusion, qui ne peut être démonté sans destruction, est intégré dans la tubulure d'entrée (12), la tubulure de sortie (13) et/ou la bride de pompe (10), la tubulure d'entrée (12), la tubulure de sortie (13) et/ou la bride de pompe (10) servant de logement au dispositif de détection d'occlusion.

10. Pompe à pistons selon la revendication 9,
**caractérisée en ce qu'**il est prévu dans le logement au moins un évidement (50) qui est fermé de façon étanche par un composant de détection (40 ; 41) qui est constitué d'un matériau sensible à la pression, le matériau du logement étant plus dur que celui du composant de détection (40 ; 41), et **en ce que** la pompe à pistons présente un détecteur de force (60) avec lequel des modifications du composant de détection (40 ; 41) provoquées par la pression dans la zone de l'évidement (50) respectif peuvent être mesurées.

11. Pompe à pistons selon la revendication 10,
**caractérisée en ce que** le raccord entre le composant de détection (40) et un logement est un raccord de moulage par injection qui a été réalisé au moyen d'un procédé à deux composants.

12. Pompe à pistons selon l'une des revendications 10 et 11 ou les deux,
**caractérisée en ce que** le composant de détection (40 ; 41) a une forme tubulaire et qu'il est installé au niveau d'une tubulure d'entrée et/ou de sortie (12 ; 13) de telle manière qu'il recouvre l'évidement (50) respectif de façon étanche depuis l'intérieur ou depuis l'extérieur.

13. Pompe à pistons selon l'une ou plusieurs des revendications 1 à 12,
**caractérisée en ce que** dans une tubulure d'entrée (12) et/ou dans une tubulure de sortie (13) est intégré respectivement un dispositif à ultrasons destiné à la détection de bulles d'air dans la tubulure respective (12 ; 13), qui ne peut être démonté sans destruction.

14. Pompe à pistons selon la revendication 13,
**caractérisée en ce que** dans la tubulure d'entrée et/ou de sortie (12 ; 13) est inséré par accouplement mécanique un tuyau (70) au moyen duquel un fluide est amené à la tubulure d'entrée (12) ou au moyen duquel un fluide est extrait à la tubulure de sortie (13), des surfaces (71 ; 72) destinées au couplage et au découplage d'ultrasons sont prévues dans la zone du tuyau (70), sur deux côtés de la tubulure d'entrée et/ou de sortie concernée (12 ; 13).

15. Dispositif d'amenée et de dosage d'un fluide à des fins médicales, **caractérisé en ce qu'**il comprend une pompe à pistons selon l'une ou plusieurs des revendications 1 à 14.
